# EUROPEAN PATENT APPLICATION

(11) **EP 3 984 442 A1**
(43) Date of publication of application: **20.04.2022**
(21) Application number: 19933001.0
(22) Date of filing: 29.07.2019
(51) Int. Cl.: A61B 3/028, A61B 3/032, A61B 3/00, A61B 3/18, E04H 1/12

(54) **PREFERENTIAL VISUAL ACUITY TEST CABIN**

(30) Priority: 13.06.2019 BR 102019012081
(71) Applicant: Rassi Gabriel, Luis Alexandre, 74140-130 Goiânia (BR)
(72) Inventor: Rassi Gabriel, Luis Alexandre, 74140-130 Goiânia (BR)
(74) Representative: Urizar Anasagasti, Jesus Maria
(86) International application number: PCT/BR2019/050302
(87) International publication number: WO 2020/248029

(57) **Abstract**

This is a booth(10), of the type intended to quantify the visual acuity, irrespective of the patient's verbal information (pc) by means of the *'Teller'* technique, in such environments as offices and clinics, by a specialized professional; said booth(10) comprises a modular structure (20), making up an isolated environment (as) for the patient (pc) to perform the preferred acuity test through the reading of *'Teller'* cards (CT), accommodated in a visualization area (21a) foreseen at the central portion of a movable wall (21) sliding by means of wheels (rd)on rails (30) and (31), installed on the ground (22) and ceiling (25) by profiles (25a); all the modular structure (20) presents a white and opaque tone, as well as its own lighting (IL), which is controlled and proper according to the test, in front of a movable wall (21) and coplanar to a rear wall (24) of booth(10); the movable wall (21) sliding proceeds the distance (d1) adjustment between the patient (pc) and the visualization area (21a)o cards (cT), where the minimum distance is equivalent to 38 centimeters and the maximum distance corresponds to 84,55 centimeters for the accuracy indicated for test effectiveness, according to the patient's (pc) features..

## Description

### TECHNICAL AREA

This patent of invention Discloses a booth for the visual acuity test, preferably in the type performed to quantify the visual acuity, irrespective of the patient's verbal information, more precisely upon the use of the "*Teller"* technique; the booth for the test of visual acuity was designed for installation in such environments as offices and clinics, and should be used by a specialized professional/ specifically, said booth comprises a structure able to provide with a more precise diagnosis, since it features a sliding means of a movable wall, which provides with the spacing adjustment between the '*Teller*' card in respect to the patient accommodated within the cabin, according to the exam specifications and the features of each patient, in addition to having the booth composing an environment all out of whatever types of visual influences liable to deviate the patient's attention, thus providing a substantial higher accuracy, dependability and reproductivity of preferred visual acuity tests.

### HISTORY OF TECHNIQUE

It is known that the visual acuity, basically deals with the eye's capacity to distinguish spatial details, i.e., to identify the contour and shape of objects, and, in such patients as children in pre-verbal phase, illiterate children with delayed neuropsychomotordevelopment, bearer of cognitive-motor, bearers of encephalic tetraplegic lesions and without speech, among others, the realization of preferred eye acuity tests is necessary, which are proper for each patient's conditions.;

The principle of visual acuity lies on the fact that human beings naturally prefer to look at something that exhibits some detail, instead of visualizing areas presenting no details, so that, upon a which board, the individual will have attention to whatever particularities present in the board, however small they might be.

One of the best used visual acuity test types is named "Teller" test, which is based on *'teller'* acuity cards, comprising rectangular plates with neutral tone, where a set of contrasting lists are presented, according to the mathematical calculation named 'cycle per centimeter' to one of the side edges of the plate. Said card further presents a central orifice that features an eye, through which the professional assesses the patient's reactions.

Upon a *"Teller"* test, the professional adopts a position to a distance that, according to the rules of such a test, might reach 84 55 or 38 cm from the patient's eyes, so that to expose the card provided with contrasting stripes turned to the patient.

Since the detail is localized solely in one of the halves of the card face turned to the patient, in case he receives the existence of such a detail, the professional will certify said fact by observing through the card orifice whether or not the patient looked at the detail. Thus, if the patient has seen the detail in the first card, one passes to the second card and so on, as he continues to look to the side containing the details. At the time the patient no longer looks at the detail, his visual acuity is noticed to be correspondent to the last card in which he was able to notice the detailing.

It happens that, in order to perform the test, the professional needs to hold the card with the hands, at the height of patient's eyes, as well it should be positioned in a way that the eye is positioned at the professional's eyes height, which leads to instability of the card.

Another inconvenient bound to the visual acuity method of *'Teller'* card verification, in the form it is currently applied, refers to the external distractions, for example, the hands of the professional, while he was sustaining the card, so that the patient deviates his look, instead of focusing the set of contracting stripes, thus making it difficult for the professional to reach a precise diagnosis in respect to the preferred visual acuity test.

### ANALYSIS ON THE CURRENT TECHNIQUE STATUS

In a research performed in specialized data bases, a document concerning the visual acuity test No CN205378090 was found, which discloses a synchronous

The utility model discloses a teller looks quick synchronous video system of card inspection arranges in by the person of examining position dead ahead eyesight board, on the eyesight board with be equipped with by the person of examining position parallel and level department leading video camera, with is watched attentively the department by the person of examining position by the person's of examining sight and be equipped with test -card locating place the eyesight backboard is equipped with video recording customer end, top to the quilt person of examining position below department and is equipped with the player of recording a video in step, the video recording customer end with record a video in step between the player through the image transmission line connection, eyesight board top orientation is equipped with the connecting rod by the position level of the person of examining position, the connecting rod tip moves towards downwards and is equipped with rearmounted video camera by the person of examining position, rearmounted video camera passes through the connecting wire and is connected with the player of recording a video in step, there are the USB interface in image transmission line and the player host computer surface in contact that records a video in step department. Only need open the video recording customer end during operation, the instruction need be carried out the infant that teller looked quick card inspection and sits at the assigned position, and this instrument can be examined all actions of infant according to inspection doctor demand in situ observation and record opening the back, and does not need other special apparatuses to help, and is not only quick convenient but also objective accurate.' (sic)

Despite it belongs to the same area of application, the document referred to the above paragraph presents none of the features of the object now improved, so ensuring that it meets the legal patentability requirements.

### OBJECTS OF THE INVENTION

An objective of this invention is to disclose a booth for preferred visual acuity test performed by *Teller* cards, wherein a booth makes up an environment for the patient to perform the preferred activity test through the reading of *'Teller'* cards, which in turn are placed within a visualization area foreseen at the central portion of a sliding movable wall on rails installed on the ground and the ceiling of the cabin, thus making it possible to adapt the distance of cards in respect to the patient's eyes and according to respective limitations.

Another objective of this invention consists in the fact that the booth composers a neutral and isolated environment for the patient to perform the acuity test, thus avoiding possible external distractions by the time of visualizing the *'Teller[* card, thus ensuring a precise diagnosis of patient's actions.

Another objective of this invention consists in the fact that the consists in the fact that the area for cards visualization, foreseen on the sliding movable wall of the card, support them in a stable form,

Another objective of this invention consists in the fact that this invention is intended to present a booth with fixed and controlled lighting, which provides a better visualization of the card, along the exam performance.

For short, the utilization of the booth provides a substantial increase of accuracy, reliability and reproductivity of preferred visual acuity tests.

### DESCRIPTION OF FIGURES

In order to complement this description, and so as to obtain a better understanding of the features of this invention, and according to a preferred practical performance of the same, an attached description follows with a set of drawings, where inits functioning was presented, in an exemplificative but not limitative form,.

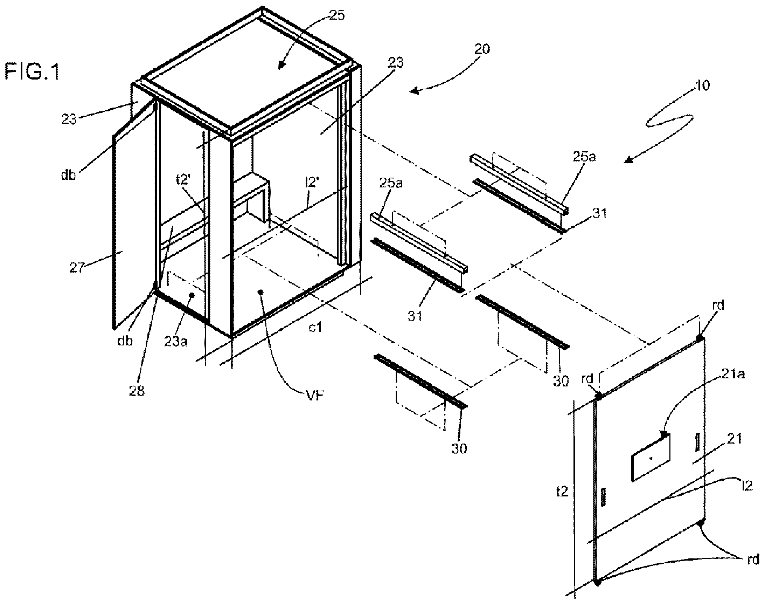

Figure 1 represents an exploded view perspective of the structure making up the mentioned booth;

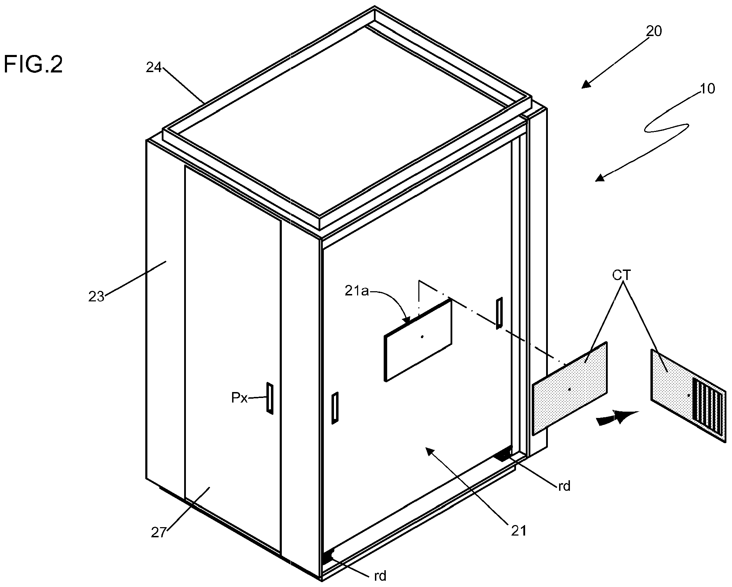

Figure 2 reveals a perspective view of the assembled cabin;

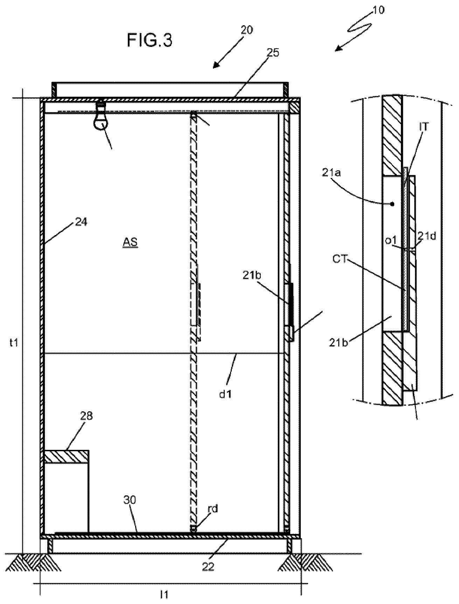

Figure 3 shows a longitudinal view AA, indicated in the former figure, in respect to the enlarged detail;

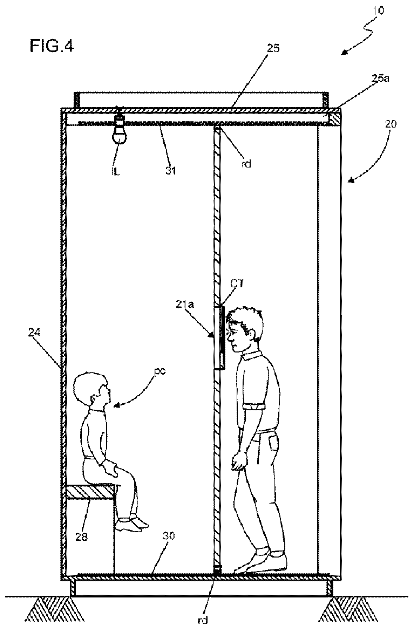

Figure 4 represents a longitudinal view AA, illustrating the sliding of the movable wall, when the proper distance adjustment for the performance of the preferred acuity test is made.

### DETAILED DESCRIPTION OF OBJECT

In respect to the illustrated drawings, this patent of invention refers to **"A BOOTH FOR THE PREFERRED VISUAL ACUITY TEST",** more precisely discloses a booth(10) of the type to quantify the visual acuity irrespective of the patient's verbal information (pc) by means of the *'teller'* technique in such environments as offices and clinics, by a specialized professional.

According to this invention, said booth(10) comprises a modular structure (20) made in wood, drywall, PVC or another type of material, in a way to make up an isolated environment (AS) for the patient (pc) to perform the preferred acuity test through the reading of 'Teller' cards (CT) laid out in the visualization area (21a) foreseen at the central portion of the sliding movable wall (21), through rollers (rd) on rails (30) and (31), installed on the ground (22) and ceiling (25), sustained by profiles (25a). All the modular structure (20) presents a white and opaque tone, as well as a proper lighting (il), which is controlled and proper, according to the test.

In front of the movable wall (21) and coplanar to the rear wall (24) of booth(10), a seat (27) is installed to accommodate the patient (pc) and an eventual companion.

The sliding of the movable wall (21) makes up the distance adjustment (d10 between the patient (pc) and the visualization area (21a) of cards (CT), where the minimum distance is equivalent to 38 centimeters and the maximum distance corresponds to 84,55 centimeters of the accuracy indicated for test effectivity, according to the patient's features (pc).

In a preferred constructive version, said modular structure (20) presents ideal height (t1), length (c1) and width (11), designed for accommodation in office, clinics or similar rooms, and comprises a floor (22), sidewalls (23), a rear wall 24) and ceiling (25), in a way to compose a frontal bay (VF) provided with a frame (26) and where a movable wall (21) is installed, with width (12) and height (t2) reduced in respect to width (I2')and eight (t2') of bay (vF). An opening (23a) is provided in one of the side walls 23), liable to receive a hinged door (27) fixed by hinges (db) and provided with a handle (Px).

The visualization area (21a) of the sliding wall (21) carts (CT) comprises a rectangular central cut-out (21b), from whose outer face a support (21c) with 'L' shape developed, mirrored in a way to cover the cut-off (21b), forming an interstice (IT) for the card (CT) assembly. Said support (21c) foresees a central hole (21d) that, upon the assembly of the card (CT), becomes aligned with the hole (o1)of the same for the professional to visualize the patient (pc) reactions who, in turn is accommodated in the booth(10).

When this invention is put into practice, it is certain that modifications concerning certain construction details and form are liable to be introduced, without implicating to deviate from the fundamental principles clearly presented in the set of claims, it being thus understood that the terminology used had no limitation purposes.

## Claims

1. "**A BOOTH FOR THE PREFERRED VISUAL ACUITY TEST**", more precisely disclosing a type of booth to quantity the visual acuity, irrespective of the verbal information about the patient (pc), by means of a *"Teller"-* type technique, such as offices and clinics by specialized professional, *featured* by a booth(10) comprising a modular environment (AS) for the patient (pc) to perform the preferred acuity test through the reading of *"Teller"* cards accommodated within a visualization area (21a), laid out in the central portion of a sliding movable wall (21) through wheels (rd) on rails (30) and (31) installed on the ground (22) and ceiling (25) sustained by profiles (25a); all the modular structure (20) presents a white and opaque tone, as well as its own lighting (II), controlled and fit according to the test; in front of the movable wall (21) and coplanar to the rear wall (24) of the booth(100) there is installed a seat (28) for accommodation of the patient (pc) and an eventual companion; the movable wall sliding (21) makes u the adjustment of distance (d1) between the patient (pc) and the visualization area (21a) of cards (C1), wherein the minimum distance, equivalent to 38 centimeters and maximum distance corresponding to 84,55 centimeters of accuracy indicated for test effectiveness, according to the patient's features (pc).

2. "**A BOOTH FOR THE PREFERRED VISUAL ACUITY TEST**" according to claim 1, *featured* by a modular structure (20), made in wood, "drywall", PVC or another type of material construction.

3. "**A BOOTH FOR THE PREFERRED VISUAL ACUITY TEST**", according to claim 1, and in a preferred construction version, *featured* by a modular structure (20) presenting ideal height (t'), length (c1) and width (11) for positioning in offices, clinics or similar rooms, and comprising a ground (22), side walls (23), a rear wall (24) and a ceiling (25), in a way to make upon a frontal bay (VIF" provided with a frame (26), where a movable wall (10) is installed, whose width (12) and height (t2) are reduced in respect to the width (I2')( and height (t2") of bay (VF); an opening (23a) is foreseen in one of the side walls (23), fixed by hinges (db) and provided with a handle (Px); the visualization area (21a) of the sliding wall (21) cards (CT) comprises a rectangular central cut-off (21b), from whose external face an 'L'-shape support 921c) is developed, in a way to cover the cut-off (21b), forming an interstice (IT) for the assembly of the card (CT), said cut-off (21b) foreseeing a central orifice (21d) which, upon the assembly of the card (CT), aligns with the orifice (o1) of the same for the professional to visualize the patient reactions (pc) who, in turn, is accommodated within the booth(10).
